# EUROPÄISCHE PATENTSCHRIFT

(11) **EP 2 630 978 B1**
(45) Veröffentlichungstag und Bekanntmachung des Hinweises auf die Patenterteilung: **31.10.2018**
(21) Anmeldenummer: 12196260.9
(22) Anmeldetag: 10.12.2012
(51) Int. Cl.: A61L 31/02, C10M 125/04

(54) **IMPLANTAT UND VERFAHREN ZUR HERSTELLUNG DESSELBEN**
IMPLANT AND METHOD FOR PRODUCTION THEREOF
IMPLANT ET SON PROCÉDÉ DE FABRICATION

(30) Priorität: 22.02.2012 US 201261601564 P
(43) Veröffentlichungstag der Anmeldung: 28.08.2013
(73) Patentinhaber: Biotronik AG, 8180 Bülach (CH)
(72) Erfinder: BAYER, Ullrich, 18211 Admannshagen-Bargeshagen (DE); BLOCK, Bernd, 18055 Rostock (DE); BECHER, Bärbel, 18057 Rostock (DE); LOOTZ, Daniel, 18119 Rostock (DE)
(74) Vertreter: Randoll, Sören

(56) Entgegenhaltungen:
- EP-A1- 2 000 551
- EP-A2- 2 172 234
- EP-A2- 2 332 588
- WO-A1-2011/051424
- US-A- 3 391 080

## Beschreibung

Die Erfindung betrifft ein Verfahren zur Herstellung eines Halbzeugs für die Herstellung eines Implantats, insbesondere einer intraluminalen Endoprothese, sowie ein Implantat und ein Halbzeug für ein solches, jeweils erhältlich oder erhalten durch ein derartiges Verfahren.

Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents, Befestigungselemente für Knochen, beispielsweise Schrauben, Platten oder Nägel, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heutzutage werden als Implantate besonders häufig Stents eingesetzt, die zur Behandlung von Stenosen (Gefäßverengungen) dienen. Sie weisen einen Körper in der Form eines rohrförmigen oder hohlzylinderförmigen Grundgitters auf, das an beiden Längsenden offen ist. Das rohrförmige Grundgitter einer derartigen Endoprothese wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können.

Ein Ansatzpunkt zur Lösung dieses Problems besteht darin, den Stent bzw. andere Implantate aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Körper biodegradierbarer Implantate geeignete Werkstoffe können beispielsweise Polymere oder Metalle enthalten. Der Körper kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Die vorliegende Erfindung bezieht sich auf Implantate mit einem metallischen biodegradierbaren Werkstoff basierend auf Magnesium oder einer Magnesiumlegierung.

Es sind bereits Stents bekannt, die Beschichtungen mit verschiedenen Funktionen aufweisen. Derartige Beschichtungen dienen beispielsweise der Freisetzung von Medikamenten, der Anordnung eines Röntgenmarkers oder dem Schutz der darunter liegenden Strukturen.

Bei der Realisierung biodegradierbarer Implantate soll die Degradierbarkeit entsprechend der angestrebten Therapie bzw. der Anwendung des jeweiligen Implantats (koronar, intracranial, renal etc.) gesteuert werden. Für viele therapeutische Anwendungen ist beispielsweise ein wichtiger Zielkorridor, dass das Implantat in einem Zeitraum von vier Wochen bis sechs Monaten seine Integrität verliert. Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, dem Aufhalten des Gefäßes, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben hat.

Als für den genannten Zielkorridor der Degradation besonders vielversprechend haben sich biodegradierbare Magnesium-Implantate, insbesondere Magnesium-Stents, erwiesen, die allerdings ihre mechanische Integrität bzw. Stützwirkung einerseits noch zu früh verlieren und andererseits in vitro und in vivo einen stark schwankenden Integritätsverlust aufweisen. Dies bedeutet, dass bei Magnesium-Stents der Kollapsdruck über die Zeit zu schnell verringert bzw. die Verringerung des Kollapsdrucks eine zu große Variabilität aufweist und damit zu unbestimmbar ist.

Im Stand der Technik sind bereits unterschiedliche Mechanismen der Degradationskontrolle von Magnesium-Implantaten beschrieben. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material, insbesondere dem metallischen Magnesium beruhen. So wird der Degradationsschutz durch verschieden zusammengesetzte Schutzschichten und durch definierte geometrische Abstände (Diffusionsbarrieren) zwischen dem Korrosionsmedium und dem Magnesiumgrundmaterial abgesichert. Weitere Lösungen auf dem Gebiet der gesteuerten Degradation rufen durch das Aufbringen von physikalischen (z.B. lokale Querschnittsveränderungen) und/oder chemischen Veränderungen der Stent-Oberfläche (z.B. lokal chemisch unterschiedlich zusammengesetzte Multilayer) Sollbrucheffekte hervor. Mit den zuvor genannten Lösungen gelingt es jedoch meist nicht, die durch den Degradationsprozess eintretende Auflösung und die daraus resultierenden Stegbrüche in das geforderte Zeitfenster zu legen. Die Folge ist eine entweder zu früh oder zu spät einsetzende bzw. eine zu große Variabilität der Degradation des Implantats.

Ein weiteres Problem im Zusammenhang mit Beschichtungen ergibt sich auch daraus, dass Stents oder andere Implantate üblicherweise zwei Zustände annehmen, nämlich einen komprimierten Zustand mit einem kleinen Durchmesser und einen expandierten Zustand mit einem größeren Durchmesser. Im komprimierten Zustand kann das Implantat mittels eines Katheters in das zu stützende Gefäß eingeführt und an der zu behandelnden Stelle positioniert werden. Am Ort der Behandlung wird das Implantat dann beispielsweise mittels eines Ballonkatheters dilatiert. Auf Grund dieser Durchmesseränderung ist der Körper des Implantats hierbei einer mechanischen Belastung ausgesetzt. Weitere mechanische Belastungen des Implantats können während der Herstellung oder bei der Bewegung des Implantats im oder mit dem Gefäß, in das das Implantat eingesetzt ist, auftreten. Bei den genannten Beschichtungen nach dem Stand der Technik ergibt sich somit der Nachteil, dass die Beschichtung während der Verformung des Implantats reißt (z.B. Bildung von Mikrorissen) oder auch teilweise entfernt wird. Hierdurch kann eine unspezifizierte lokale Degradation verursacht werden. Außerdem sind das Einsetzen und die Geschwindigkeit der Degradation von der Größe und der Verteilung der durch die Verformung entstehenden Mikrorisse abhängig, die als Fehlstellen schlecht kontrollierbar sind. Dies führt zu einer starken Streuung in den Degradationszeiten.

Weitere Beispiele für bekannte organische oder anorganische Schutzschichten zur Erhöhung der Degradationsbeständigkeit sind galvanische Beschichtungen mittels Zink, Beschichtungen auf der Basis ionischer Flüssigkeiten, Konversionsschichten durch chemische Umwandlung der Hauptlegierungsbestandteile, Bedampfen oder Sputtern mit Aluminium, thermisches Spritzen und dergleichen.

Ferner werden höher legierte Ausgangsmaterialien zur Verbesserung der Degradationsbeständigkeit verwendet. So werden seit Jahren Seltene Erden enthaltende Magnesiumlegierungen (z.B. die Legierung WE 43) als Stentmaterial eingesetzt. Diese Materialien weisen für viele Anwendungsfälle eine vorteilhafte Kombination aus hinreichenden mechanischen Eigenschaften und guter Korrosionsbeständigkeit auf. Diese haben dadurch im Vergleich zu unlegiertem Magnesium ein größeres Anwendungspotential für absorbierbare Implantate.

Aus der Druckschrift WO 2011/051424 A1 ist ein implantierbares medizinisches Gerät bekannt, das zumindest teilweise aus einem Material besteht, das hochreines Magnesium oder eine Magnesiumlegierung mit hochreinem Magnesium und ein oder mehrere weitere hochreine Legierungsbestandteile enthält. Hochreine Legierungsbestandteile können die Elemente Indium, Scandium, Yttrium, Gallium und die Seltenen Erden darstellen, wobei hochreines Gallium mit 0,1 Gew.% bis 5 Gew.% in der Legierung vorhanden ist. Die Herstellung von derartigen hochreinen Materialien ist sehr teuer, so dass auch die Kosten für das medizinische Gerät hoch sind.

EP2000551 A1 beschreibt Stents auf Basis von Magnesium. Auch EP 2332588 A2 beschreibt solche Implantate. US 3391080 beschreibt die Anwendung von Gallium in der Luftfahrttechnik. EP 2172234 A2 offenbart Implantate.

In der Druckschrift WO 2007/0207186 A1 sind eine Reihe von Additiven, welche die Eigenschaften eines Implantats verbessern, genannt. Hierbei werden jedoch die geänderten Eigenschaften des Implantats hinsichtlich des jeweiligen Additivs nicht im Einzelnen erläutert.

Folglich besteht die Aufgabe der vorliegenden Erfindung darin, ein kostengünstiges Verfahren zur Herstellung eines Implantats anzugeben, bei dem die Degradation des Implantats verzögerter verläuft. Dabei soll die Degradation in einem besser kontrollierbaren Zeitraum stattfinden. Entsprechend besteht die Aufgabe der Erfindung außerdem darin, ein derartiges Implantat zu schaffen.

Die obige Aufgabenstellung wird gelöst durch ein Verfahren zur Herstellung eines Halbzeugs für die Herstellung eines Implantats, wobei das Halbzeug Magnesium oder eine Magnesiumlegierung enthält, umfassend die folgenden Schritte:
a) Bereitstellen des Halbzeugs
b) Umformen des Halbzeugs bei einer Temperatur zwischen 250°C und 550°C mittels eines Werkzeugs, das zumindest auf einem Teil seiner mit dem Halbzeug in Berührung kommenden Oberfläche ein metallisches Schmiermittel mit Gallium und/oder mit einer Galliumverbindung (einschließlich Galliumlegierung) aufweist.

Bei der vorliegenden Erfindung erfolgt das Umformen bei einer Temperatur über 300°C, bevorzugt über 350°C. Die Obergrenze für die Umformtemperatur liegt bei etwa 550°C, da oberhalb von 550°C Gefügeteile des Implantats schmelzflüssig werden und gegebenenfalls Verluste hinsichtlich der mechanischen Festigkeit beim Endprodukt (Implantat) auftreten können.

Mit dem nach dem erfindungsgemäßen Verfahren verwendeten Schmiermittel wird einerseits die, vorzugsweise für das Umformverfahren Extrusion, erforderliche Minimierung der Reibung zwischen dem umzuformenden Werkstoff und dem Werkzeug erzielt. Das Schmiermittel ist oberhalb der Raumtemperatur flüssig und besitzt durch seine Viskosität eine geringe Scherfestigkeit, so dass eine gute Schmierwirkung erzielt wird. Weiter vorteilhaft ist, dass in dem Temperaturbereich das Schmiermittel nur einen geringen Dampfdruck hat und nur eine unwesentliche Viskositätsänderung aufweist.

Das Schmiermittel weist neben der Schmierfunktion zudem die Besonderheit auf, dass es während des Umformens, insbesondere während eines Extrusionsprozesses, in die Oberfläche des Halbzeugs eindiffundiert und sich mit diesem legiert. Die hohe Diffusionsgeschwindigkeit, die bei den zur Anwendung kommenden erhöhten Temperaturen auftritt, führt zu einer Legierungsbildung an den Oberflächen des Halbzeugs bereits während der Prozesszeit. Die Tiefe und die stöchiometrische Zusammensetzung des auflegierten Oberflächenbereichs (oberflächennaher Bereich, Oberflächenzone) werden durch die Prozessparameter des Umformprozesses (z.B. die Umformgeschwindigkeit, die Temperatur, die Schmiermittelmenge, die Schmiermittelzusammensetzung (z.B. bei einer Extrusion)) und eine nachfolgende Temperbehandlung bestimmt, welche in ihrer Temperatur und ihrer Zeitdauer variieren kann. Das im Schmiermittel dominierende Element Gallium führt durch die Legierungsbildung mit Magnesium zu einem veränderten Implantat hinsichtlich seiner mechanischen und biochemischen Eigenschaften, die unten im Einzelnen beschrieben werden.

Das Schmiermittel hat somit multifunktionale Eigenschaften.

Das in dem erfindungsgemäßen Verfahren verwendete Schmiermittel wird anstelle der konventionellen Schmiermittel Graphit oder Molybdändisulfid eingesetzt, welche nicht rückstandsfrei von der Halbzeugoberfläche oder den oberflächennahen Bereichen entfernt werden können. Zudem reagieren diese herkömmlichen Schmiermittel bei den üblichen Prozessparametern insbesondere während der Extrusion auf unerwünschte Weise mit der Oberfläche des magnesiumhaltigen Grundmaterials und bilden fest anhaftende Krusten, die aus Verbindungen der in diesen Schmiermitteln enthaltenden Elemente, beispielsweise C, O, Mo, S, mit Magnesium bestehen. Diese Krusten müssen aufwändig während des nachfolgenden Prozesses bei der Herstellung des Implantats beseitigt werden, z.B. mittels Reibahlen, Korund-Strahlen, Beizen und/oder Elektropolieren. Da bei dem erfindungsgemäßen Verfahren jedoch diese Krusten nicht entstehen, sind auch die genannten Beseitigungsschritte nicht erforderlich, so dass die Herstellung des Implantats auch aus diesem Grund kostengünstig und einfach ist.

Aus dem genannten Halbzeug kann dann auf einfache Weise ein Implantat hergestellt werden, das einen absorbierbaren Gradientenwerkstoff aufweist und dass sich durch eine Reihe von Vorteilen gegenüber Implantaten aus konventionellem Werkstoff hinsichtlich seines Degradationsverhaltens auszeichnet.

Das Implantat weist verbesserte mechanische Eigenschaften, z.B. ein besseres Umformverhalten, und eine verbesserte dynamische Belastbarkeit durch eine verminderte Anfälligkeit gegenüber oberflächenfehler-induzierten Rissen bei dynamischer Beanspruchung auf. Insbesondere ist ein solches Implantat beim Dilatieren (z.B. beim Dilatieren eines Stents) weniger bruchanfällig. So wurde gefunden, dass der mittlere Durchmesser des ersten Stegbruchs beim Dilatieren von Stents eines spezifischen Designs von 4,6 mm (Referenz unbehandelt) auf 4,9 mm (erfindungsgemäße Lösung) angehoben werden konnte.

Ferner wird durch eine Veränderung des elektrochemischen Oberflächenpotentials durch das Gallium die Degradationsgeschwindigkeit in Richtung längerer Degradationszeiten verändert, wobei die gewünschte geringere Degradationsgeschwindigkeit durch die Änderung der Diffusionstiefe und/oder die Legierungszusammensetzung eingestellt werden kann.

Außerdem kann die Degradationsgeschwindigkeit durch Variation der Schichtdicke gesteuert werden. Hierdurch eröffnet sich auch die Möglichkeit, die Degradationsdauer des Implantats an den spezifischen Implantationsort anzupassen (koronar, intercranial, renal etc.). Ferner wurde beobachtet, dass sich der Zeitraum der Degradation in vorteilhafter Weise von vorher 4 bis 6 Monate (unbeschichtete Referenz) auf nunmehr 7 bis 8 Monate (erfindungsgemäße Lösung) verlängert. Weiterhin können Kosten im Herstellungsprozess des Implantats gespart werden, da das Halbzeug besser weiter verarbeitbar ist, weil Schlackeanhaftungen, die z.B. beim Laserschneiden des Implantats an der Schnittkante entstehen, leichter entfernt werden können.

Das Halbzeug weist zudem gegenüber bekannten Halbzeugen, wenn es nach den erfindungsgemäßen Verfahren hergestellt wird, eine verbesserte Oberflächenqualität auf, so dass bei einer nachfolgenden Elektropolitur ein gleichmäßigerer Abtrag der Oberflächenschicht entsteht. Ggf. bedingt die verbesserte Oberflächenqualität, dass außerdem eine dauerhafte Anhaftung bzw. eine gleichmäßige Beschichtung der Oberfläche mit Polymeren erfolgen kann.

Dem gegenüber zeigen konventionell, z.B. mittels Plattierverfahren, hergestellte Halbzeuge bei der Verformung der daraus gefertigten Endprodukte, beispielsweise beim Dilatieren eines Stens, häufig Delaminationen einer aufgebrachten Schicht vom Grundkörper.

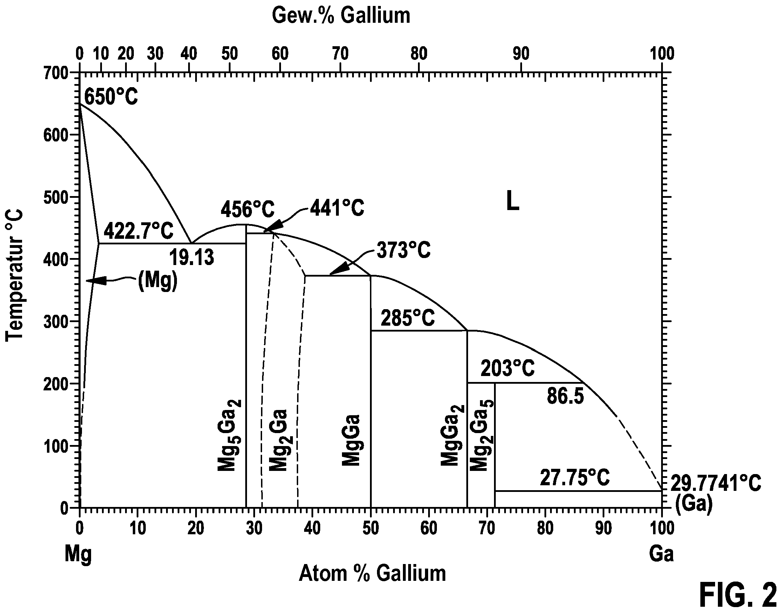

Während des Umformschritts diffundiert das Schmiermittel, das zwischen Werkzeugwand und Halbzeug angeordnet ist, in die Magnesium-Oberfläche des Halbzeugs hinein. Wie das in Figur 2 dargestellte Temperatur-Zeit-Diagramm des Systems Magnesium/Gallium zeigt, besitzt das System Magnesium-Gallium ein Eutektikum bei 423°C und 19 Atom% Gallium und es existiert eine maximale Löslichkeit etwa 8 Gew.% Gallium in Magnesium bei 423°C. Eine derartige Löslichkeit zeigt, dass eine Legierung aus Magnesium und Gallium gebildet werden kann. Das nicht einlegierte, überflüssige Gallium bzw. Schmiermittel kann nach dem Umformprozess sowohl vom Werkzeug als auch vom Halbzeug abgestreift werden.

Ein weiterer Vorteil des mit dem erfindungsgemäßen Verfahren hergestellten Implantats besteht darin, dass diese eine verbesserte Biokompatibilität aufweisen, da die chemische Oberflächenzusammensetzung auf der luminalen und der abluminalen Seite des Implantats hinsichtlich des jeweiligen Gewebes bzw. die Körperflüssigkeit eingestellt werden kann.

In einem bevorzugten Ausführungsbeispiel beinhaltet das Umformen einen Extrusionsschritt, der in einem geeigneten Temperaturbereich für die Löslichkeit des Galliums im Magnesium durchgeführt wird.

Ein besonders geeignetes Schmiermittel für das erfindungsgemäße Verfahren ist Galinstan®, welches eine silberne, eutektische Legierung aus Gallium, Indium und Zinn darstellt. Galinstan® ist bei Raumtemperatur flüssig und geht laut Angaben des Herstellers Geratherm Medical AG, Geschwenda, Deutschland bei Temperaturen unter 19°C in den festen Aggregatzustand über. Galinstan® besteht zu 65 bis 95 Gew.% aus Gallium, zu 5 bis 22 Gew.% aus Indium und zu 0 bis 11 Gew.% aus Zinn.

Für das Einlegieren des Galliums ist es von Vorteil, wenn das Umformen zumindest zeitweise, vorzugsweise über mindestens 30 Sekunden, bei einer Temperatur von mindestens 300°C durchgeführt wird. Bei Prozesstemperaturen von mindestens 300°C entstehen Mischphasen von Magnesium und Gallium, die sich laut binärem Phasendiagramm in Abhängigkeit der Prozesshaltezeit von mehreren Minuten bis in Tiefen von 40 µm ausbilden. Das dabei aus Magnesium-Gallium Mischkristallen bestehende Gefüge zeichnet sich gegenüber einer rein metallischen Magnesium-Matrix durch einen erhöhten korrosiven Widerstand und eine erhöhte Bruchzähigkeit aus.

Des weiteren wird durch die Eigenschaft, dass aufgrund von Diffusionsprozessen die Konzentration des Galliums von der Halbzeug-Oberfläche in die Tiefe des Werkstoffs abnimmt, eine "Verzahnung" des galliumreichen oberflächennahen Bereichs des Halbzeugs und des Grundmaterials (ohne Gallium) des Halbzeugs erreicht, so dass bei dem hieraus hergestellten Implantat in vorteilhafter Weise keine Delaminationen der oberflächennahen galliumreichen Schicht bei Biege-, Scher- und Torsionsbeanspruchung bei Einsatzbedingungen des Implantats auftreten.

In einer Weiterbildung der Erfindung wird nach dem Umformen ein Temperschritt bei mindestens 300°C über mindestens 1 Minute durchgeführt und das Halbzeug anschließend vorzugsweise an Luft und vorzugsweise bei Raumtemperatur abgekühlt. Dieser Temperschritt dient dazu, in dem Gefüge nach dem Umformen enthaltene innere mechanische Spannungen abzubauen. Falls dieser Spannungsarmglühprozess nicht durchgeführt werden würde, käme es zu unerwünschten geometrischen Verformungen des Halbzeuges, die sich negativ auf die folgenden Verarbeitungsschritte auswirken würden (z.B. Taumeln in der Laserspannvorrichtung und damit verbundene erhöhte Schneidungenauigkeit beim Laserschneiden).

Als weitere Bearbeitungsschritte bis zur Fertigstellung des Implantats schließen sich das Entgraten, Beizen und/oder Elektropolieren an. Diese Schritte können zu einer Reduzierung der Schichtdicke der mit Gallium angereicherten Randschicht führen.

Die obige Aufgabe wird außerdem durch ein Halbzeug für die Herstellung eines Implantats gelöst, wobei das Halbzeug Magnesium oder eine Magnesiumlegierung aufweist, erhältlich oder erhalten durch eines der oben erläuterten Verfahren.

Entsprechend wird obige Aufgabe auch durch ein Implantat mit einem Implantatkörper gelöst, wobei der Implantatkörper Magnesium oder eine Magnesiumlegierung aufweist, erhältlich oder erhalten durch eines der oben angegebenen Verfahren.

Die obige Aufgabe wird auch durch ein Implantat, insbesondere eine intraluminalen Endoprothese, gelöst, mit einem Körper, wobei der Körper Magnesium oder eine Magnesiumlegierung und in einem oberflächennahen Bereich Gallium und/oder eine Galliumlegierung enthält.

Für das erfindungsgemäße Implantat ist von Vorteil, dass nach den obigen erfindungsgemäßen Verfahren das Schmiermittel mit Gallium oder einer Galliumverbindung einerseits die erforderliche Minimierung der Reibung zwischen dem umzuformenden Werkstoff und dem Werkzeug erreicht wird. Andererseits besteht in einem oberflächennahen Bereich des Halbzeugs bzw. des Implantats eine Schicht, in der Gallium (aus dem Schmiermittel) ein Legierungsbestandteil für das bereits in dem Halbzeug bzw. Implantatkörper vorliegenden Magnesiums bildet. Das überschüssige, nicht an der Legierungsbildung beteiligte Gallium kann von der Oberfläche des Halbzeugs einfach entfernt werden. Neben den oben bereits genannten Vorteilen weist ein aus dem Halbzeug hergestelltes Implantat eine erhöhte Schadenstoleranz beim Vorhandensein von inneren Kerben, beispielsweise hervorgerufen durch Einschlüsse und Stringer aus intermetallischen Verbindungen des Magnesium mit legierungstypischen Elementen auf.

Dieser Effekt beruht auf der höheren plastischen Verformbarkeit galliumreicher Körner des Implantatgefüges, die bei mechanischer Beanspruchung in der Lage sind, weitere Gleitebenen zu aktivieren. Laufen also von inneren Kerben herrührende Anrisse in die mit Gallium auflegierten Körner des Implantatgefüges hinein, kommt es zunächst zu einer plastischen Verformung der Körner und Ausbildung von Gleitbändern. Demgegenüber wandeln sich in einem Implantat, dessen Halbzeug während des Herstellungsprozesses nicht mit einem galliumhaltigen Schmiermittel umgeformt wird, Anrisse zu einem mikroskopischen Riss um, so dass ein früher Stegbruch resultiert.

Der oberflächennahe Bereich mit dem Gallium bzw. der Galliumverbindung in dem resultierenden Implantat weist vorzugsweise eine Dicke von mindestens 10 µm, besonders bevorzugt von mindestens 15 µm, weiter bevorzugt von maximal 40 µm auf.

Der Körper des Implantats umfasst mindestens einen Teil des Implantats, vorzugsweise den Hauptteil des Implantats, welcher die mechanische Integrität des Implantats bewirkt. Die vorliegende Erfindung bezieht sich dabei insbesondere auf Implantate, deren Material des Körpers Magnesium als Hauptbestandteil enthält und vorzugsweise biodegradierbar ist.

In einem weiteren bevorzugten Ausführungsbeispiel wird der Implantat-Körper nach dem Laserschneiden und Entgraten elektrochemisch behandelt, vorzugsweise elektrochemisch poliert. Hierdurch werden Verunreinigungen auf der Oberfläche des Implantat-Körpers entfernt. Das Elektropolieren führt zu kontaminationsarmen Deckschichten auf Grund großer Materialabtragseffekte (Tiefenwirkung).

Das erfindungsgemäße Verfahren wird nachfolgend anhand von Beispielen und Figuren erläutert. Dabei bilden alle abgebildeten und/oder beschriebenen Merkmale den Gegenstand der Erfindung, unabhängig von ihrer Zusammenfassung in den Ansprüchen oder deren Rückbeziehung.

Es zeigen schematisch:
- Fig. 1: einen Querschnitt durch ein erfindungsgemäßes Halbzeug (beispielsweise für einen Stent) nach Schritt b) des erfindungsgemäßen Verfahrens und
- Fig. 2: das binäre Zustandsdiagramm für das System Magnesium-Gallium.

Zunächst soll darauf hingewiesen werden, dass die in der nachfolgenden Beschreibung enthaltenen Angaben zu Konzentrationen eines Materials, sofern an der betreffenden Stelle nichts Anderes vermerkt ist, jeweils in Masse% angegeben sind.

Eine röhrenförmige oder hohlzylinderförmige Hülse aus einer Magnesiumlegierung, beispielsweise mit der Zusammensetzung WE 43 (4% Y, 2% Nd, 0,5% Gd, 0,5% Dy, 0,5% Zr, Rest Mg) wird zur Herstellung eines Implantat-Halbzeugs, beispielsweise für einen Stent, einem Extrusionsprozess unterzogen. Vor der Extrusion wird auf die Oberfläche des Extrusionswerkzeugs Galinstan® mit einer beispielhaften Zusammensetzung von 68,5% Ga, 21,5% In und 10% Sn als Schmiermittel dort aufgetragen, wo die Oberfläche des Extrusionswerkzeugs mit der Hülse in Kontakt gelangt. Dabei kann der die Innengeometrie bestimmende Stempel und/oder die die spätere Außenoberfläche bestimmende Matrize mit dem Schmiermittel versehen werden.

Das galliumhaltige Schmiermittel wird vor dem Extrusionsprozess auf das Außenwerkzeug (Stempel) und/oder in das Innenwerkzeug (Matrize) tropfenförmig aufgebracht. Danach werden die Werkzeuge und der auf dem Stempel angeordnete umzuformende Rohling (Abmessungen z.B. Außendurchmesser 3 mm bis 10 mm, Innendurchmesser 1 mm bis 5 mm, Länge 3 mm bis 15 mm) auf die Prozesstemperatur aufgeheizt, welche zwischen 300°C und 500°C liegt. Der Aufheizvorgang dauert ca. 1 Minute bis 10 Minuten. Danach erfolgt der eigentliche Umformprozess, bei dem der auf dem Stempel angeordnete zylinderförmige Rohling in der Matrize zu einem Halbzeug 10 in Form einer Hülse (Abmessungen z.B. Außendurchmesser 2 mm, Innendurchmesser 1,6 mm, Länge 50 mm bis 200 mm) extrudiert wird. Die Pressgeschwindigkeit kann beispielsweise im Bereich zwischen 1 mm/min bis 100 mm/min betragen, so dass der Extrusionsvorgang nach ca. 30 Sekunden bis 200 Minuten abgeschlossen ist.

Mit dem Einführen des Stempels in den in der Matrize angeordneten Rohling (auch Butze genannt) und dem vorausgehenden Aufheizen der Hülse auf die Prozesstemperatur in Höhe von etwa 400°C entfaltet Gallium zunächst seine Wirkung als Schmiermittel aufgrund seiner im schmelzflüssigen Zustand ausgeprägten, äußerst geringen Scherkräfte.

Während des Umformens diffundieren die Elemente des galliumhaltigen Schmiermittels in die entstehende neue Innen- und/oder Außenoberfläche des Halbzeugs 10 hinein und beginnen, mit dem Magnesium eine Legierung zu bilden.

Das extrudierte Halbzeug 10 wird anschließend dem Werkzeug entnommen und kühlt innerhalb weniger Minuten auf nahezu Raumtemperatur ab.

Danach erfolgt ein Temperschritt, vorzugsweise bei 300°C bis 500°C, zur Beseitigung von Spannungen (sogenanntes Spannungsarmglühen) über einen Zeitraum von 1 Minute bis 60 Minuten an Luft oder unter Inertgas wie Argon. In den oberflächennahen Zonen finden dabei zwei Vorgänge statt. Zum Einen schreitet die Diffusion der Elemente des Schmiermittels in das Volumen des Halbzeugs fort, zum Anderen kommt es zur Bildung einer Legierung aus Mg und Ga. Hierbei lagert sich entweder das Gallium teilweise im Mg-Gitter als Substituent entsprechend des in Fig. 2 gezeigten binären Zustandsdiagramms Mg-Ga an oder es kommt zur Bildung von intermetallischen Verbindungen, z.B. Mg₅Ga₂, Mg₂Ga und MgGa, in einem oberflächennahen Bereich 12 (bzw. Schicht). Hierdurch entstehen Mischphasen, die sich laut Phasendiagramm (siehe Fig. 2) in Abhängigkeit von der Prozesshaltezeit von mehreren Minuten bis in eine Tiefe von bis zu 40 µm ausbilden. Durch Veränderung der vorstehend genannten Prozessparameter Temperatur und Zeit kann die Diffusionstiefe des Gallium in das Halbzeug in der Größenordnung zwischen 20 µm (bei 450°C/10 min) und 40 µm (470°C/40 min) gemessen von der jeweiligen Oberfläche des Halbzeugs variiert werden.

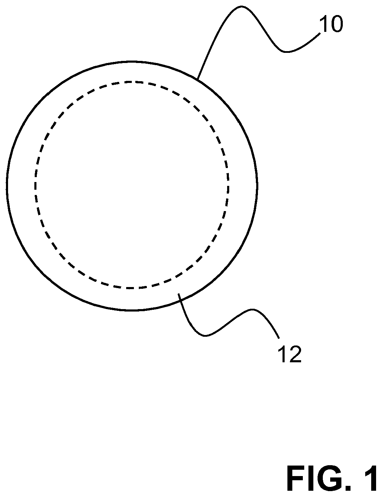

Fig. 1 zeigt die erhöhte Galliumkonzentration in dem oberflächennahen Grenzbereich 12 lediglich an der abluminalen Seite. Entsprechend der Auftragung des Schmiermittels in dem Werkzeug kann alternativ oder zusätzlich die luminale Seite des Halbzeugs einen solchen oberflächennahen Grenzbereich mit erhöhter Galliumkonzentration aufweisen

Eine Überdosierung an Schmiermittel ist nicht möglich, da überschüssiges Schmiermittel bei Prozessende entweder am Werkzeug verbleibt oder von diesem abgestreift wird.

Die weiteren Prozessschritte gestalten sich analog zu den bekannten und vielfach beschriebenen Verfahren der Herstellung von Implantaten mittels Laserstrahlverfahren und umfassen finaler Formgebungsprozesse wie Laserschneiden, Entgraten, Beizen und Elektropolieren. Besonders zu erwähnen ist in diesem Zusammenhang, dass die Beiz- und Elektropolierschritte in phosphorsäurehaltigen Lösungen bei Raumtemperatur über Zeiträume zwischen 0,5 Minuten und 4 Minuten (bevorzugt 2 Minuten) ablaufen. Das Beizen und Elektropolieren ist mit Materialabtrag verbunden. Beispielsweise beträgt der Materialabtrag bei einem Beizen über 2 Minuten zwischen 10 µm und 20 µm der Wanddicke. Somit weist das Implantat, z.B. der Stent, am Ende des Herstellungsverfahrens noch eine Wanddicke zwischen 140 µm und 170 µm auf, wobei die Wanddicke des Halbzeugs nach dem Extrudieren und Tempern und vor dem Elektropolieren und/oder Beizen 180 µm bis 190 µm beträgt. Bei Anwendung der beschriebenen Prozessparameter ist jedoch sichergestellt, dass die Diffusionstiefe des Galliums im Magnesiummatrixmaterial größer ist als der durch den Beiz- und/oder Polierschritt auftretende Materialabtrag.

Auch das fertige Implantat weist analog zum Halbzeug 10 in einem oberflächennahen Grenzbereich der Außen- und/oder Innenseite seiner Stege eine erhöhte Gallium-Konzentration auf.

Es ist von Vorteil, dass in dem Halbzeug 10 durch die auf metallurgischem Wege hergestellte "Verzahnung" des an Gallium reichen oberflächennahen Bereichs mit dem Grundmaterial erfolgt ist. Dies bedeutet, dass in dem fertigen Implantat keine Delaminationserscheinungen bei Biege-, Scher- oder Torsionsbeanspruchung unter Einsatzbedingungen des Implantats auftreten.

Wie bereits oben erwähnt wurde, wird das binäre Zustandsdiagramm für das System Gallium-Magnesium in Fig. 2 dargestellt, wobei auf der X-Achse die Gallium-Konzentration in Gew.-% bzw. Atom-% und auf der Y-Achse die Temperatur in °C aufgetragen sind. Mit dem Buchstabe L ist der Liquidusbereich gekennzeichnet.

### Bezugszeichenliste

- 10: Halbzeug
- 12: oberflächennaher Bereich
- L: Liquidusbereich

## Patentansprüche

1. Verfahren zur Herstellung eines Halbzeugs (10) für die Herstellung eines Implantats aus einem Halbzeug, wobei das Halbzeug Magnesium oder eine Magnesiumlegierung enthält, umfassend die folgenden Schritte:
a) Bereitstellen des Halbzeugs,
b) Umformen des Halbzeugs bei einer Temperatur über 300°C und bis 550°C mittels eines Werkzeugs, das zumindest auf einem Teil seiner mit dem Halbzeug in Berührung kommenden Oberfläche ein metallisches Schmiermittel mit Gallium und/oder mit einer Galliumverbindung aufweist.

2. Verfahren nach Anspruch 1, **dadurch gekennzeichnet, dass** das Umformen einen Extrusionsschritt beinhaltet.

3. Verfahren nach einem der vorhergehenden Ansprüche **dadurch gekennzeichnet, dass** das Schmiermittel Galinstan enthält.

4. Verfahren nach einem der vorhergehenden Ansprüche, **dadurch gekennzeichnet, dass** das Umformen mindestens über 30 Sekunden, bei einer Temperatur von mindestens 300°C durchgeführt wird.

5. Verfahren nach einem der vorhergehenden Ansprüche, **dadurch gekennzeichnet, dass** nach dem Umformen ein Temperschritt bei mindestens 300°C über mindestens 1 Minute durchgeführt und das Halbzeug anschließend vorzugsweise an Luft und vorzugsweise bei Raumtemperatur abgekühlt wird.

6. Halbzeug (10) für die Herstellung eines Implantats erhältlich durch eines der in den vorhergehenden Ansprüchen angegebenen Verfahren.

7. Implantat mit einem Implantatkörper aus einem Halbzeug (10), wobei das Halbzeug (10) durch eines der in den Ansprüchen 1 bis 5 angegebenen Verfahren hergestellt wurde.

## Claims

1. A method for producing a semifinished article (10) for producing an implant from a semifinished article, wherein the semifinished article contains magnesium or a magnesium alloy, said method comprising the following steps:
a) preparing the semifinished article,
b) shaping the semifinished article at a temperature above 300°C and up to 550°C using a tool, which has a metallic lubricant containing gallium and/or a gallium compound on at least a part of its surface that will come into contact with the semifinished article.

2. The method according to claim 1, **characterized in that** the shaping comprises an extrusion step.

3. The method according to any one of the preceding claims, **characterized in that** the lubricant contains Galinstan.

4. The method according to any one of the preceding claims, **characterized in that** the shaping is carried out at least over a period of 30 seconds, at a temperature of at least 300°C.

5. The method according to any one of the preceding claims, **characterized in that** after the shaping process, a tempering step is carried out at least at 300°C over a period of at least 1 minute, after which the semifinished article is preferably air cooled, preferably at room temperature.

6. A semifinished article (10) for the production of an implant which is obtainable by one of the methods specified in the preceding claims.

7. An implant having an implant body of a semifinished article (10), wherein the semifinished article (10) was prepared by one of the methods specified in claims 1 to 5.

## Revendications

1. Procédé de confection d'un produit semi-fini (10) destiné à la fabrication d'un implant à partir du produit semi-fini, le produit semi-fini contenant du magnésium ou un alliage de magnésium, comprenant les étapes suivantes :
a) de mise à disposition du produit semi-fini,
b) de façonnage du produit semi-fini à une température au-dessus de 300 °C et jusqu'à 550 °C au moyen d'un outil qui présente au moins sur une partie de sa surface venant en contact avec le produit semi-fini un lubrifiant avec du gallium et/ou avec un composé de gallium.

2. Procédé selon la revendication 1, **caractérisé en ce que** le façonnage contient une étape d'extrusion.

3. Procédé selon l'une des revendications précédentes, **caractérisé en ce que** le lubrifiant contient du galinstan.

4. Procédé selon l'une des revendications précédentes, **caractérisé en ce que** le façonnage est effectué pendant au moins 30 secondes à une température d'au moins 300 °C.

5. Procédé selon l'une des revendications précédentes, **caractérisé en ce qu'**après le façonnage, une étape de thermostatation à au moins 300 °C pendant au moins 1 minute est effectuée et le produit semi-fini est ensuite refroidi, de préférence à l'air et de préférence à température ambiante.

6. Produit semi-fini (10) destiné à la fabrication d'un implant confectionné par un procédé indiqué dans les revendications précédentes.

7. Implant avec un corps d'implant à base d'un produit semi-fini (10), le produit semi-fini ayant été fabriqué par un procédé indiqué dans les revendications 1 à 5.
